# EUROPEAN PATENT SPECIFICATION

(11) **EP 1 582 232 B1**
(45) Date of publication and mention of the grant of the patent: **03.01.2007**
(21) Application number: 05006994.7
(22) Date of filing: 31.03.2005
(51) Int. Cl.: A61M 39/22, A61M 5/168

(54) **Valve for use in implantable infusion pumps**
Ventil zur Verwendung in implantierbaren Infusionspumpen
Soupape pour utilisation dans pompes d'infusion implantables

(30) Priority: 03.04.2004 DE 102004016443
(43) Date of publication of application: 05.10.2005
(73) Proprietor: Codman Neuro Sciences Sàrl, 2400 Le Locle (CH)
(72) Inventor: Wieland, Manfred, 24146 Kiel (DE); Bauman, Hans, Dr., 24223 Raisdorf (DE); Kulig, Manfred, Dipl.-Ing., 24647 Ehndorf (DE); Zacharias, Volker, Dr., 24106 Kiel (DE)
(74) Representative: Heselberger, Johannes

(56) References cited:
- EP-A- 0 387 439
- EP-A- 0 763 368
- WO-A-01/66173
- WO-A-92/16247
- US-A- 5 277 556
- US-A1- 2003 100 863

## Description

### BACKGROUND OF THE INVENTION

### 1. Field of the Invention

The present invention relates to a valve. More specifically, the present invention relates to a valve for use in implantable infusion pumps.

### 2. Discussion of Related Art

Valves of various embodiments are known for a long time. Valves have an inlet, a valve body interacting with a valve seat, an actuating element acting upon the valve body and on outlet. An example of a prior art valve is disclosed in "Brockhaus, Naturwissenschaften und Technik", 1983, Bd. 5, S. 192.

It is an object of the present invention to create a compact and reliable valve, which is suitable for the conveyance of very small quantities of liquid. It is also an object of the present invention to provide a valve that if it fails, will do so in a safe state. It is a further object to provide a valve that has a compact design, which would be well suited for an implantable medical device. It is a further object of the present invention to provide a valve that has a minimal displacement, thereby allowing for minimal energy consumption. It is a further object of the present invention to provide a valve that has a minimal dead volume.

### SUMMARY OF THE INVENTION

According to the present invention these and other objects are solved in an exemplary embodiment of a valve having a housing, an inlet, an outlet and a ram receiving chamber. A ram has a first end and a second end and is slidably supported in the ram receiving chamber. The ram has a central pin on the second end of the ram. An actuating element is connected to the ram. A plate is fixedly connected to the housing. The plate has a central bore. The ram and plate selectively abut to form a valve seat. The inlet is in fluid communication with the ram receiving chamber. The ram has an outer wall. Fluid communicates between a portion of the ram receiving chamber adjacent to the first end of the ram and a portion of the ram receiving chamber adjacent to the second end of the ram. In the closed position, the ram abuts with the plate. The pin protrudes through the central bore in the plate. In the open position, the actuating element displaces the ram such that the ram is spaced from the plate.

### BRIEF DESCRIPTION OF THE DRAWING FIGURES

Figure 1 is a sectional view of a valve in accordance with an exemplary embodiment of the present invention, shown in the closed position; and
Figure 2 is a sectional view of the valve of Figure 1, shown in the open position.

### DETAILED DESCRIPTION OF THE PRESENT INVENTION

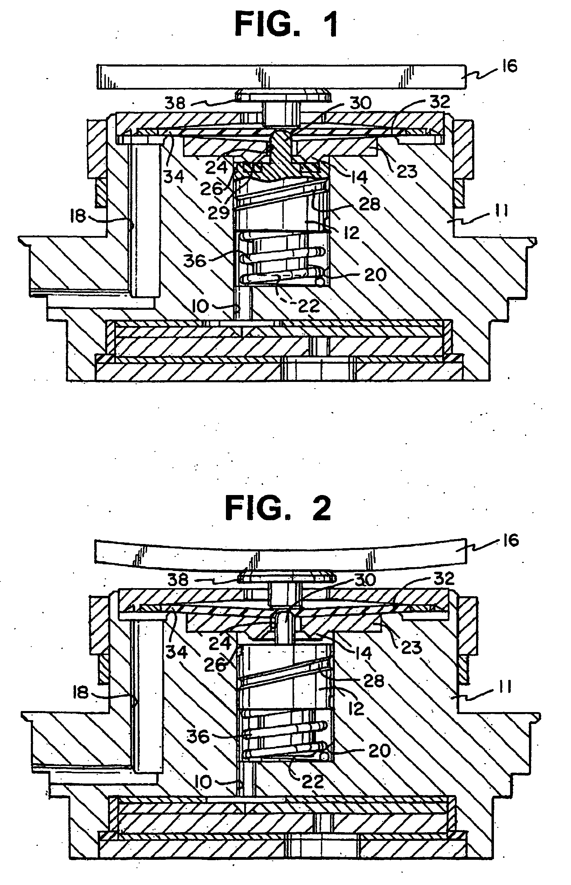

Referring now to Figures 1 and 2, the valve includes a cylindrical ram 12 forming the valve body. Ram 12 is axially slidably supported within a cylindrical ram receiver 20, which is in the form of a bore in a valve housing 11. An inlet 10 is in fluid communication with one axial end 22, sometimes referred to as the lower face, of ram 12. The other axial end 26 of ram 12 is sometimes referred to as the upper face.

A groove 28 is formed in the outer wall of ram 12. Groove 28 provides fluid communication between the chamber adjacent to the lower face 22 within the housing 11 and the chamber adjacent to the upper face 26. Groove 28 is in a currently preferred exemplary embodiment a spiral groove. But groove 28 could be axial. Additionally, ram 12 could have multiple grooves, such as two, three or even more grooves. Alternatively, ram 12 could have no grooves, but a relatively small annular gap would exist between the outer circumferential wall of ram 12 and the inner cylindrical wall of cam receiving chamber 20. In another alternative design, the groove or grooves could be formed in the inner cylindrical wall of the cam receiving chamber 20 and not in the outer surface of the ram. In addition, the groove or grooves could be formed in the inner cylindrical wall of the cam receiving chamber 20 and in the outer surface of ram 12. Thus, in any embodiment, liquid entering via inlet 10 flows to the area of the cam receiving chamber adjacent to lower face 22 of the ram 12 and is conveyed from this area to the area of the cam receiving chamber adjacent to upper face 26 of the ram 12 via groove(s) 28 or via the annular gap between the outer circumferential wall of ram 12 and the inner cylindrical wall of cam receiving chamber 20.

A gasket 29 is formed in the upper face 26 of ram 12. In a currently preferred exemplary embodiment, gasket 29 is made of an elastically yielding plastic material, preferably silicone. But gasket 29 could be made of any biocompatible deformable plastic or soft metal. A plate 23 is fixedly connected to housing 11. Plate 23 includes a downwardly depending annular projection 14 that forms a valve seat for gasket 29 of ram 12. As illustrated in Figure 1, the valve is in the closed position. Thus, gasket 29 abuts against projection 14 to prevent fluid from travelling past the valve seat to an outlet 18 of the valve. Plate 23 has a central recess or bore 24, thereby forming an annular channel between bore 24 and an upwardly projecting pin 30 of ram 12. Pin 30 protrudes through bore 24 of plate 23. In the open position illustrated in Figure 2, ram 12 is displaced downwards such that the gasket 29 is spaced from the projection 14. Thus, the liquid can flow past the valve seat from the outer area above front face 26 to the inner area above front face 26 and through the annular channel between bore 24 and pin 30. The fluid then flows through a gap 34 between a plate 23 and a membrane 32 to the outlet 18 and out of the valve housing 11.

In a currently preferred exemplary embodiment, projection 14 has a trapezoidically shape in cross-section as illustrated. But projection 14 could have different shapes, such as, for example, hemispherical. In another embodiment projection 14 could be formed on the front face 26 or ram 12 and gasket 29 could be formed in plate 23.

Central pin 30 is provided on the front face 26 and is directed towards plate 23. An actuating element 16, preferably in the form of a bending plate, acts upon the central pin 30. Actuating element 16 has a downwardly projecting projection 38, which is aligned with pin 30. A flexible membrane 32 is disposed between the free end of the pin 30 and the free end of the projection 38 of actuating element 16. Membrane 32 forms a hermetic seal within the housing. Thus, the fluid within the valve housing 11 doesn't contact projection 38 or actuating element 16. In a currently preferred exemplary embodiment, actuating element 16 is a piezoelectric element, which requires very little energy to move from the position illustrated in the drawing Figure to the downward position, thereby opening the valve. In addition, in a currently preferred exemplary embodiment, membrane 32 is made of titanium. But membrane 32 could be made of stainless steel. In addition, membrane 32 could have a bellows shape.

A coil spring 36 rests within the ram receiver 20 and acts upon the lower face 22 of ram 12. Thus, spring 36 biases ram 12 in the upper direction to the position illustrated in the drawing, thereby closing the valve.

Even if the valve were to fail, the valve would be brought to the illustrated closed position due to the coil spring 36 acting on ram 12. Additionally, the fluid flowing into the valve will apply a pressure on the entire lower face 22 of ram 12, but only on an outer annular portion of upper face 26. The fluid will naturally bias ram 12 upwardly against projection 14 of plate 23. Thus, if the valve in accordance with the present invention fails, it will do so in a safe state. This is especially important to prevent unwanted volume of fluids from being infused into the body.

The valve in accordance with the present invention has a compact design, which is well suited for an implantable medical device, such as, for example, an implantable infusion pump. Within such a pump, the pump inlet is in fluid communication with the valve inlet, and the pump outlet is in fluid communication with the valve outlet. The valve has a minimal displacement, thereby allowing for minimal energy consumption. The valve also has a minimal dead volume.

In the specification, terms, such as, for example, "upper", "lower", "upwardly" and "downwardly", etc. are used with reference to the drawing figures to ease the description of the present invention. However, these terms are not to be so limited as the valve could be oriented in virtually any direction. Thus, for example, the term "upwardly" may not necessarily be upward when the valve is used.

## Claims

1. A valve comprising:
a housing having an inlet, an outlet and a ram receiving chamber;
a ram having a first end and a second end, the ram is slidably supported in the ram receiving chamber, the ram has a central pin on the second end of the ram;
an actuating element being connected to the ram;
a plate being fixedly connected to the housing, the plate has a central bore, the ram and the plate selectively abut to form a valve seat.;
wherein the inlet is in fluid communication with the ram receiving chamber , the ram has an outer wall, a portion of the ram receiving chamber adjacent to the first end of the ram is in fluid communication with a portion of the ram receiving chamber adjacent to the second end of the ram; in the closed position the ram abuts with the plate ; the pin protrudes through the central bore in the plate, in the open position the actuating element displaces the ram such that the ram is spaced from the plate.

2. The valve according to claim 1, wherein the actuating element is a piezoelectrical bending plate.

3. The valve according to claim 1, wherein the ram receiving chamber is cylindrical.

4. The valve according to claim 1, further comprising a flexible membrane connected to the housing and disposed between a free end of the pin and the actuating element.

5. The valve according to claim 4, wherein the membrane is made of titanium.

6. The valve according to claim 4, wherein the membrane forms a hermetic seal within the housing.

7. The valve according to claim 4, wherein a gap is disposed between the plate and the membrane, the gap is in fluid communication with the outlet.

8. The valve according to claim 7, wherein the gap is annular.

9. The valve according to claim 1, further comprising a spring disposed within the ram receiving chamber, the ram is biased by the spring into the closed position.

10. The valve according to claim 9, wherein the spring is a coil spring, the spring acts upon the first end of the ram.

11. The valve according to claim 1, wherein the actuating element has a projection that is axially aligned with the central pin.

12. The valve according to claim 1, wherein the ram is cylindrical in shape.

13. The valve according to claim 12, wherein the ram is displaced axially.

14. The valve according to claim 1, wherein the ram has a gasket on the second end of the ram.

15. The valve according to claim 14, wherein the plate has a depending projection, the gasket and the depending projection selectively abut to form the valve seat.

16. The valve according to claim 15, wherein the depending projection has a trapezoidal shape in cross-section.

17. The valve according to claim 14, wherein the gasket is made of an elastically yielding plastic material.

18. The valve according to claim 1, wherein the ram has a groove disposed in its outer wall.

19. An implantable infusion pump comprising:
a housing having a pump inlet, a pump outlet
a valve having a housing, an inlet fluidly connected to the pump inlet, and an outlet fluidly connected to the pump outlet, the valve having a ram receiving chamber;
a ram having a first end and a second end, the ram is slidably supported in the ram receiving chamber, the ram has a central pin on the second end of the ram;
an actuating element being connected to the ram;
a plate being fixedly connected to the housing, the plate has a central bore, the ram and the plate selectively abut to form a valve seat;
wherein the inlet is in fluid communication with the ram receiving chamber, the ram has an outer wall, a portion of the ram receiving chamber adjacent to the first end of the ram is in fluid communication with a portion of the ram receiving chamber adjacent to the second end of the ram; in the closed position the ram abuts with the plate; the pin protrudes through the central bore in the plate, in the open position the actuating element displaces the ram such that the ram is spaced from the plate.

## Patentansprüche

1. Ein Ventil, welches aufweist:
ein Gehäuse mit einem Einlass, einem Auslass und einer Kolben-aufnehmenden Kammer;
einen Kolben mit einem ersten Ende und einem zweiten Ende, wobei der Kolben gleitend unterstützt wird in der Kolben-aufnehmenden Kammer, wobei der Kolben auf dem zweiten Ende des Kolbens einen zentralen Stift aufweist;
ein Betätigungselement, welches mit dem Kolben verbunden ist;
eine Platte, welche fest verbunden ist mit dem Gehäuse, wobei die Platte eine zentrale Bohrung aufweist und wobei der Kolben und die Platte sich selektiv berühren, um einen Ventilsitz zu bilden;
wobei der Einlass in Fluidkommunikation mit der Kolben-aufnehmenden Kammer ist, wobei der Kolben eine äußere Wand aufweist, ein Teil der Kolben-aufnehmenden Kammer neben dem ersten Ende des Kolbens in Fluidkommunikation mit einem Teil der Kolben-aufnehmenden Kammer neben dem zweiten Ende des Kolbens ist; in der geschlossenen Position berührt der Kolben die Platte; der Stift ragt durch die zentrale Bohrung in der Platte hervor und wobei in der offenen Position das Betätigungselement den Kolben so verschiebt, dass der Kolben von der Platte beabstandet ist.

2. Das Ventil gemäß Anspruch 1, wobei das Betätigungselement eine piezoelektrische Biegeplatte ist.

3. Das Ventil gemäß Anspruch 1, wobei die Kolben-aufnehmende Kammer zylindrisch ist.

4. Das Ventil gemäß Anspruch 1, weiterhin aufweisend eine flexible Membran, welche mit dem Gehäuse verbunden ist und zwischen einem freien Ende des Stifts und dem Betätigungselement angeordnet ist.

5. Das Ventil gemäß Anspruch 4, wobei die Membran aus Titan hergestellt ist.

6. Das Ventil gemäß Anspruch 4, wobei die Membran eine hermetische Abdichtung innerhalb des Gehäuses bildet.

7. Das Ventil gemäß Anspruch 4, wobei eine Lücke zwischen der Platte und der Membran angeordnet ist, wobei die Lücke in Fluidkommunikation mit dem Auslass ist.

8. Das Ventil gemäß Anspruch 7, wobei die Lücke ringförmig ist.

9. Das Ventil gemäß Anspruch 1, weiterhin aufweisend eine Feder, welche innerhalb der Kolben-aufnehmenden Kammer angeordnet ist, wobei der Kolben vorgespannt wird durch die Feder in die geschlossene Position.

10. Das Ventil gemäß Anspruch 9, wobei die Feder eine Spiralfeder ist, wobei die Feder auf das erste Ende des Kolbens einwirkt.

11. Das Ventil gemäß Anspruch 1, wobei das Betätigungselement einen Vorsprung aufweist, welcher axial mit dem zentralen Stift ausgerichtet ist.

12. Das Ventil gemäß Anspruch 1, wobei der Kolben eine zylindrische Form aufweist.

13. Das Ventil gemäß Anspruch 12, wobei der Kolben axial verschoben ist.

14. Das Ventil gemäß Anspruch 1, wobei der Kolben einen Dichtungsring auf dem zweiten Ende des Kolbens aufweist.

15. Das Ventil gemäß Anspruch 14, wobei die Platte einen zugehörigen Vorsprung aufweist, wobei der Dichtungsring und der zugehörige Vorsprung sich selektiv berühren, um den Ventilsitz zu bilden.

16. Das Ventil gemäß Anspruch 15, wobei der zugehörige Vorsprung im Querschnitt eine trapezartige Form aufweist.

17. Das Ventil gemäß Anspruch 14, wobei der Dichtungsring aus einem elastisch nachgiebigen Plastikmaterial gebildet ist.

18. Das Ventil gemäß Anspruch 1, wobei der Kolben eine Nut aufweist, welche in seiner äußeren Wand angeordnet ist.

19. Eine implantierbare Infusionspumpe, welche aufweist:
ein Gehäuse mit einem Pumpeneinlass, einem Pumpenauslass;
ein Ventil mit einem Gehäuse, einem Einlass, welcher flüssig verbunden ist mit dem Pumpeneinlass und einem Auslass, welcher flüssig verbunden ist mit dem Pumpenauslass, wobei das Ventil eine Kolben-aufnehmende Kammer aufweist;
einen Kolben mit einem ersten Ende und einem zweiten Ende, wobei der Kolben in der Kolben-aufnehmenden Kammer gleitend unterstützt wird, wobei der Kolben einen zentralen Stift auf dem zweiten Ende des Kolbens aufweist;
ein Betätigungselement, welches mit dem Kolben verbunden ist;
eine Platte, welches fest verbunden ist mit dem Gehäuse, wobei die Platte eine zentrale Bohrung aufweist, und wobei der Kolben und die Platte sich selektiv berühren, um einen Ventilsitz zu bilden;
wobei der Einlass in Fluidkommunikation mit der Kolben-aufnehmenden Kammer ist, der Kolben eine äußere Wand aufweist, ein Teil der Kolben-aufnehmenden Kammer neben dem ersten Ende des Kolbens in Fluidkommunikation mit einem Teil der Kolben-aufnehmenden Kammer neben dem zweiten Ende des Kolbens ist; in der geschlossenen Position berührt der Kolben die Platte; der Stift ragt durch die zentrale Bohrung in der Platte hervor, und wobei die offene Position des Betätigungselements den Kolben so verschiebt, dass der Kolben von der Platte beabstandet ist.

## Revendications

1. Soupape, comprenant :
un boîtier comportant un passage d'entrée, un passage de sortie et une chambre de logement de piston-plongeur ;
un piston-plongeur présentant une première extrémité et une seconde extrémité, le piston-plongeur étant soutenu, de manière coulissante, dans la chambre de logement de piston-plongeur, le piston-plongeur comportant un axe central sur la seconde extrémité du piston-plongeur ;
un élément d'actionnement, relié au piston-plongeur ;
une plaque, fixée au boîtier, la plaque comportant un alésage central, et le piston-plongeur et la plaque venant sélectivement en butée l'un contre l'autre pour former un siège de soupape ;
dans laquelle le passage d'entrée est en communication de fluide avec la chambre de logement de piston-plongeur, le piston-plongeur présente une paroi extérieure, une partie de la chambre de logement de piston-plongeur, adjacente à la première extrémité du piston-plongeur, est en communication de fluide avec une partie de la chambre de logement de piston-plongeur, adjacente à la seconde extrémité du piston-plongeur ; dans laquelle, dans la position fermée, le piston-plongeur vient en butée contre la plaque, l'axe faisant saillie au-delà de l'alésage central ménagé dans la plaque et, dans la position ouverte, l'élément d'actionnement déplace le piston-plongeur, qui est alors écarté de la plaque.

2. Soupape selon la revendication 1, dans laquelle l'élément d'actionnement est une plaque piézo-électrique à flexion.

3. Soupape selon la revendication 1, dans laquelle la chambre de logement de piston-plongeur est cylindrique.

4. Soupape selon la revendication 1, comprenant en outre une membrane flexible, reliée au boîtier et disposée entre une extrémité libre de l'axe et l'élément d'actionnement.

5. Soupape selon la revendication 4, dans laquelle la membrane est faite en titane.

6. Soupape selon la revendication 4, dans laquelle la membrane forme une fermeture hermétique à l'intérieur du boîtier.

7. Soupape selon la revendication 4, dans laquelle un interstice est prévu entre la plaque et la membrane, l'interstice étant en communication de fluide avec le passage de sortie.

8. Soupape selon la revendication 7, dans laquelle l'interstice est annulaire.

9. Soupape selon la revendication 1, comprenant en outre un ressort, disposé à l'intérieur de la chambre de logement de piston-plongeur, le piston-plongeur étant sollicité par le ressort pour prendre la position fermée.

10. Soupape selon la revendication 9, dans laquelle le ressort est un ressort en spirale, le ressort agissant sur la première extrémité du piston-plongeur.

11. Soupape selon la revendication 1, dans laquelle l'élément d'actionnement comporte une protubérance, qui est en alignement axial avec l'axe central.

12. Soupape selon la revendication 1, dans laquelle le piston-plongeur est de forme cylindrique.

13. Soupape selon la revendication 12, dans laquelle le piston-plongeur est déplacé axialement.

14. Soupape selon la revendication 1, dans laquelle le piston-plongeur comporte un joint d'étanchéité sur sa seconde extrémité.

15. Soupape selon la revendication 14, dans laquelle la plaque présente une protubérance d'un seul tenant, le joint d'étanchéité et la protubérance d'un seul tenant venant sélectivement en butée pour former le siège de soupape.

16. Soupape selon la revendication 15, dans laquelle la protubérance d'un seul tenant a une section transversale de forme trapézoïdale.

17. Soupape selon la revendication 14, dans laquelle le joint d'étanchéité est fait en un matériau plastique à déformation élastique.

18. Soupape selon la revendication 1, dans laquelle le piston-plongeur comporte une gorge, située dans sa paroi extérieure.

19. Pompe implantable d'infusion, comprenant :
un carter de pompe comportant un passage d'entrée de pompe, un passage de sortie de pompe ;
une soupape comportant un boîtier, un passage d'entrée en communication de fluide avec le passage d'entrée de pompe, et un passage de sortie en communication de fluide avec le passage de sortie de pompe, la soupape comprenant une chambre de logement de piston-plongeur ;
un piston-plongeur présentant une première extrémité et une seconde extrémité, le piston-plongeur étant soutenu, de manière coulissante, dans la chambre de logement de piston-plongeur, le piston-plongeur comportant un axe central sur la seconde extrémité du piston-plongeur ;
un élément d'actionnement, relié au piston-plongeur ;
une plaque, fixée au boîtier, la plaque comportant un alésage central, et le piston-plongeur et la plaque venant sélectivement en butée l'un contre l'autre pour former un siège de soupape ;
dans laquelle le passage d'entrée est en communication de fluide avec la chambre de logement de piston-plongeur, le piston-plongeur présente une paroi extérieure, une partie de la chambre de logement de piston-plongeur, adjacente à la première extrémité du piston-plongeur, est en communication de fluide avec une partie de la chambre de logement de piston-plongeur, adjacente à la seconde extrémité du piston-plongeur ; dans laquelle, dans la position fermée, le piston-plongeur vient en butée contre la plaque, l'axe faisant saillie au-delà de l'alésage central ménagé dans la plaque et, dans la position ouverte, l'élément d'actionnement déplace le piston-plongeur, qui est alors écarté de la plaque.
